# EUROPEAN PATENT SPECIFICATION

(11) **EP 3 126 505 B1**
(45) Date of publication and mention of the grant of the patent: **16.10.2019**
(21) Application number: 15716121.7
(22) Date of filing: 01.04.2015
(51) Int. Cl.: C12N 15/86, C12N 15/09, A61K 39/285

(54) **ONCOLYTIC VACCINIA VIRUS**
ONKOLYTISCHES VACCINIA-VIRUS
VIRUS DE LA VACCINE ONCOLYTIQUE

(30) Priority: 01.04.2014 GB 201405834
(43) Date of publication of application: 08.02.2017
(73) Proprietor: Queen Mary University of London, London E1 4NS (GB)
(72) Inventor: WANG, Yaohe, London EC1M 6BQ (GB); YUAN, Ming, London EC1M 6BQ (GB); AHMED, Jahangir, London EC1M 6BQ (GB); LEMOINE, Nicholas Robert, London EC1M 6BQ (GB)
(74) Representative: Bassil, Nicholas Charles
(86) International application number: PCT/GB2015/051023
(87) International publication number: WO 2015/150809

(56) References cited:
- CN-A- 103 110 939
- C T HILEY ET AL: "Lister strain vaccinia virus, a potential therapeutic vector targeting hypoxic tumours", GENE THERAPY, vol. 17, no. 2, 1 February 2010 (2010-02-01), pages 281-287, XP055194542, ISSN: 0969-7128, DOI: 10.1038/gt.2009.132
- J. DIMIER ET AL: "Deletion of Major Nonessential Genomic Regions in the Vaccinia Virus Lister Strain Enhances Attenuation without Altering Vaccine Efficacy in Mice", JOURNAL OF VIROLOGY, vol. 85, no. 10, 15 May 2011 (2011-05-15), pages 5016-5026, XP055194226, ISSN: 0022-538X, DOI: 10.1128/JVI.02359-10
- BARTLETT N ET AL: "The vaccinia virus N1L protein is an intracellular homodimer that promotes virulence", JOURNAL OF GENERAL VIROLOGY, SOCIETY FOR GENERAL MICROBIOLOGY, SPENCERS WOOD, GB, vol. 83, no. 8, 15 July 2002 (2002-07-15), pages 1965-1976, XP002409054, ISSN: 0022-1317 cited in the application
- BILLINGS B ET AL: "Lack of N1L gene expression results in a significant decrease of vaccinia virus replication in mouse brain", ANNALS OF THE NEW YORK ACADEMY OF SCIENCES, NEW YORK ACADEMY OF SCIENCES, US, vol. 1030, 1 December 2004 (2004-12-01), pages 297-302, XP009086080, ISSN: 0077-8923, DOI: 10.1196/ANNALS.1329.037
- A. POSTIGO ET AL: "The Vaccinia Virus-Encoded Bcl-2 Homologues Do Not Act as Direct Bax Inhibitors", JOURNAL OF VIROLOGY, vol. 86, no. 1, 1 January 2012 (2012-01-01), pages 203-213, XP055194591, ISSN: 0022-538X, DOI: 10.1128/JVI.05817-11
- AHMED JAHANGIR ET AL: "A Novel Tumour Selective Oncolytic Vaccinia Virus Lacking the N1L Gene Enhances the Anti-Tumour Immune Response When Used as an Anticancer Therapeutic", MOLECULAR THERAPY, vol. 22, no. Suppl. 1, May 2014 (2014-05), pages S248-S249, XP002740900, & 17TH ANNUAL MEETING OF THE AMERICAN-SOCIETY-OF-GENE-AND-CELL-THERAPY (ASGCT); WASHINGTON, DC, USA; MAY 21 -24, 2014

### SEQUENCE LISTING

<110> Queen Mary University of London
<120> Oncolytic virus
<130> P58571WO NCB
<140> PCT/GB2015/051023
   <141> 2015-04-01
<150> GB 1405834.1
   <151> 2014-04-01
<160> 7
<170> PatentIn version 3.3
<210> 1
   <211> 70
   <212> DNA
   <213> Artificial sequence
<220>
   <223> Synthetic sequence: Modified vaccinia promoter mH5
<400> 1
   aaaaattgaa aataaataca aaggttcttg agggttgtgt taaattgaaa gcgagaaata      60
   atcataaata                                                              70
<210> 2
   <211> 4102
   <212> DNA
   <213> Artificial sequence
<220>
   <223> Synthetic sequence: Expression cassette comprising three mH5 promoters
<400> 2
   tttggttata ctagtatagt cactatcgga caaataaaga aaatcagatg atcgatgaat      60
   aatacattta aattcatcat ctgtaagatt tttgagatgt ctcattagaa tattattagg     120
   gttagtactc attatcattc ggcagctatt acttatttta ttatttttca ccatatagat     180
   caatcattag atcaaaaatt gaaaataaat acaaaggttc ttgagggttg tgttaaattg     240
   aaagcgagaa ataatcataa atagtcgaca atcgaattcc cgcggccggg aattcgatta     300
   gatctaaaaa ttgaaaataa atacaaaggt tcttgagggt tgtgttaaat tgaaagcgag     360
   aaataatcat aaatagctac cggactcaga tccaccggtc gccaccatgg cctcctccga     420
   ggacgtcatc aaggagttca tgcgcttcaa ggtgcgcatg gagggctccg tgaacggcca     480
   cgagttcgag atcgagggcg agggcgaggg ccgcccctac gagggcaccc agaccgccaa     540
   gctgaaggtg accaagggcg gccccctgcc cttcgcctgg gacatcctgt ccccccagtt     600
   ccagtacggc tccaaggtgt acgtgaagca ccccgccgac atccccgact acaagaagct     660
   gtccttcccc gagggcttca agtgggagcg cgtgatgaac ttcgaggacg gcggcgtggt 720
   gaccgtgacc caggactcct ccctgcagga cggctccttc atctacaagg tgaagttcat 780
   cggcgtgaac ttcccctccg acggccccgt aatgcagaag aagactatgg gctgggaggc 840
   ctccaccgag cgcctgtacc cccgcgacgg cgtgctgaag ggcgagatcc acaaggccct 900
   gaagctgaag gacggcggcc actacctggt ggagttcaag tccatctaca tggccaagaa 960
   gcccgtgcag ctgcccggct actactacgt ggactccaag ctggacatca cctcccacaa 1020
   cgaggactac actatcgtgg agcagtacga gcgcgccgag ggccgccacc acctgttcct 1080
   gtagcggccg cgactctaga tcataatcag ccataccaca tttgtagagg ttttacttgc 1140
   tttaaaaaac ctcccacacc tccccctgaa cctgaaacat aaaatgaatg caattgttgt 1200
   tgttaacttg tttattgcag cttataatgg ttacaaataa agcaatagca tcacaaattt 1260
   cacaaataaa gcattttttt cactgcattc tagttgtggt ttgtccaaac tcatcaatgt 1320
   atcttaaggc gaaaaattga aaataaatac aaaggttctt gagggttgtg ttaaattgaa 1380
   agcgagaaat aatcataaat aaagcttcga ggggctcgca tctctccttc acgcgcccgc 1440
   cgccctacct gaggccgcca tccacgccgg ttgagtcgcg ttctgccgcc tcccgcctgt 1500
   ggtgcctcct gaactgcgtc cgccgtctag gtaagtttaa agctcaggtc gagaccgggc 1560
   ctttgtccgg cgctcccttg gagcctacct agactcagcc ggctctccac gctttgcctg 1620
   accctgcttg ctcaactcta cgtctttgtt tcgttttctg ttctgcgccg ttacagatcc 1680
   aagctgtgac cggcgcctac gtaagtgata tctactagat ttatcaaaaa gagtgttgac 1740
   ttgtgagcgc tcacaattga tacttagatt catcgagagg gacacgtcga ctactaacct 1800
   tcttctcttt cctacagctg agatcaccgg cgaaggaggg ccaccatggg tcaccagcag 1860
   ttggtcatct cttggttttc cctggttttt ctggcatctc ccctcgtggc catatgggaa 1920
   ctgaagaaag atgtttatgt cgtagaattg gattggtatc cggatgcccc tggagaaatg 1980
   gtggtcctca cctgtgacac ccctgaagaa gatggtatca cctggacctt ggaccagagc 2040
   agtgaggtct taggctctgg caaaaccctg accatccaag tcaaagagtt tggagatgct 2100
   ggccagtaca cctgtcacaa aggaggcgag gttctaagcc attcgctcct gctgcttcac 2160
   aaaaaggaag atggaatttg gtccactgat attttaaagg accagaaaga acccaaaaat 2220
   aagacctttc taagatgcga ggccaagaat tattctggac gtttcacctg ctggtggctg 2280
   acgacaatca gtactgattt gacattcagt gtcaaaagca gcagaggctc ttctgacccc 2340
   caaggggtga cgtgcggagc tgctacactc tctgcagaga gagtcagagg ggacaacaag 2400
   gagtatgagt actcagtgga gtgccaggag gacagtgcct gcccagctgc tgaggagagt 2460
   ctgcccattg aggtcatggt ggatgccgtt cacaagctca agtatgaaaa ctacaccagc 2520
   agcttcttca tcagggacat catcaaacct gacccaccca agaacttgca gctgaagcca 2580
   ttaaagaatt ctcggcaggt ggaggtcagc tgggagtacc ctgacacctg gagtactcca 2640
   cattcctact tctccctgac attctgcgtt caggtccagg gcaagagcaa gagagaaaag 2700
   aaagatagag tcttcacgga caagacctca gccacggtca tctgccgcaa aaatgccagc 2760
   attagcgtgc gggcccagga ccgctactat agctcatctt ggagcgaatg ggcatctgtg 2820
   ccctgcagtg ttcctggagt aggggtacct ggggtgggcg ccagaaacct ccccgtggcc 2880
   actccagacc caggaatgtt cccatgcctt caccactccc aaaacctgct gagggccgtc 2940
   agcaacatgc tccagaaggc cagacaaact ctagaatttt acccttgcac ttctgaagag 3000
   attgatcatg aagatatcac aaaagataaa accagcacag tggaggcctg tttaccattg 3060
   gaattaacca agaatgagag ttgcctaaat tccagagaga cctctttcat aactaatggg 3120
   agttgcctgg cctccagaaa gacctctttt atgatggccc tgtgccttag tagtatttat 3180
   gaagacttga agatgtacca ggtggagttc aagaccatga atgcaaagct gctgatggat 3240
   cctaagaggc agatctttct agatcaaaac atgctggcag ttattgatga gctgatgcag 3300
   gccctgaatt tcaacagtga gactgtgcca caaaaatcct cccttgaaga accggatttt 3360
   tataaaacta aaatcaagct ctgcatactt cttcatgctt tcagaattcg ggcagtgact 3420
   attgatagag tgatgagcta tctgaatgct tcctaaaaag cgaggtccct ccaaaccgtt 3480
   gtcattttta taaaactttg aaatgaggaa actttgatag gatgtggatt aagaactagg 3540
   gaggggctag ctcgacatga taagatacat tgatgagttt ggacaaacca caactagaat 3600
   gcagtgaaaa aaatgcttta tttgtgaaat ttgtgatgct attgctttat ttgtgaaatt 3660
   tgtgatgcta ttgctttatt tgtaaccatt ataagctgca ataaacaagt taacaacaac 3720
   aattgcattc attttatgtt tcaggttcag ggggaggtgt gggaggtttt ttaaagcaag 3780
   taaaacctct acaaatgtgg tagatccatt tgggacgcgt atctaataag tagagtcctc 3840
   atgcttagtt aacaactatt ttttatgtta aatcaattag tacaccgcta tgtttaatac 3900
   ttattcatat tttagttttt aggattgaga atcaatacaa aaattaatgc atcattaatt 3960
   ttagaaatac ttagtttcca cgtagtcaat gaaacatttg aactcatcgt acaggacgtt 4020
   ctcgtacagg acgtaactat aaaccggttt atatttgttc aagatagata caaatccgat 4080
   aacttttttt acgaattcta cg 4102
<210> 3
   <211> 155
   <212> PRT
   <213> Homo sapiens
<400> 3
   Met Glu Arg Ile Val Ile Cys Leu Met Val Ile Phe Leu Gly Thr Leu
   1               5                   10                  15
   Val His Lys Ser Ser Ser Gln Gly Gln Asp Arg His Met Ile Arg Met
               20                  25                  30
   Arg Gln Leu Ile Asp Ile Val Asp Gln Leu Lys Asn Tyr Val Asn Asp
           35                  40                  45
   Leu Val Pro Glu Phe Leu Pro Ala Pro Glu Asp Val Glu Thr Asn Cys
       50                  55                  60
   Glu Trp Ser Ala Phe Ser Cys Phe Gln Lys Ala Gln Leu Lys Ser Ala
   65                  70                  75                  80
   Asn Thr Gly Asn Asn Glu Arg Ile Ile Asn Val Ser Ile Lys Lys Leu
                   85                  90                  95
   Lys Arg Lys Pro Pro Ser Thr Asn Ala Gly Arg Arg Gln Lys His Arg
               100                 105                 110
   Leu Thr Cys Pro Ser Cys Asp Ser Tyr Glu Lys Lys Pro Pro Lys Glu
           115                 120                 125
   Phe Leu Glu Arg Phe Lys Ser Leu Leu Gln Lys Met Ile His Gln His
       130                 135                 140
   Leu Ser Ser Arg Thr His Gly Ser Glu Asp Ser
   145                 150                 155
<210> 4
   <211> 146
   <212> PRT
   <213> Homo sapiens
<400> 4
   Met Glu Arg Ile Val Ile Cys Leu Met Val Ile Phe Leu Gly Thr Leu
   1               5                   10                  15
   Val His Lys Ser Ser Ser Gln Gly Gln Asp Arg His Met Ile Arg Met
               20                  25                  30
   Arg Gln Leu Ile Asp Ile Val Asp Gln Leu Lys Asn Tyr Val Asn Asp
           35                  40                  45
   Leu Val Pro Glu Phe Leu Pro Ala Pro Glu Asp Val Glu Thr Asn Cys
       50                  55                  60
   Glu Trp Ser Ala Phe Ser Cys Phe Gln Lys Ala Gln Leu Lys Ser Ala
   65                  70                  75                  80
   Asn Thr Gly Asn Asn Glu Arg Ile Ile Asn Val Ser Ile Lys Lys Leu
                   85                  90                  95
   Lys Arg Lys Pro Pro Ser Thr Asn Ala Gly Arg Arg Gln Lys His Arg
               100                 105                 110
   Leu Thr Cys Pro Ser Cys Asp Ser Tyr Glu Lys Lys Pro Pro Lys Glu
           115                 120                 125
   Phe Leu Glu Arg Phe Lys Ser Leu Leu Gln Lys Val Ser Thr Leu Ser
       130                 135                 140
   Phe Ile
   145
<210> 5
   <211> 219
   <212> PRT
   <213> Homo sapiens
<400> 5
   Met Cys Pro Ala Arg Ser Leu Leu Leu Val Ala Thr Leu Val Leu Leu
   1               5                   10                  15
   Asp His Leu Ser Leu Ala Arg Asn Leu Pro Val Ala Thr Pro Asp Pro
               20                  25                  30
   Gly Met Phe Pro Cys Leu His His Ser Gln Asn Leu Leu Arg Ala Val
           35                  40                  45
   Ser Asn Met Leu Gln Lys Ala Arg Gln Thr Leu Glu Phe Tyr Pro Cys
       50                  55                  60
   Thr Ser Glu Glu Ile Asp His Glu Asp Ile Thr Lys Asp Lys Thr Ser
   65                  70                  75                  80
   Thr Val Glu Ala Cys Leu Pro Leu Glu Leu Thr Lys Asn Glu Ser Cys
                   85                  90                  95
   Leu Asn Ser Arg Glu Thr Ser Phe Ile Thr Asn Gly Ser Cys Leu Ala
               100                 105                 110
   Ser Arg Lys Thr Ser Phe Met Met Ala Leu Cys Leu Ser Ser Ile Tyr
           115                 120                 125
   Glu Asp Leu Lys Met Tyr Gln Val Glu Phe Lys Thr Met Asn Ala Lys
       130                 135                 140
   Leu Leu Met Asp Pro Lys Arg Gln Ile Phe Leu Asp Gln Asn Met Leu
   145                 150                 155                 160
   Ala Val Ile Asp Glu Leu Met Gln Ala Leu Asn Phe Asn Ser Glu Thr
                   165                 170                 175
   Val Pro Gln Lys Ser Ser Leu Glu Glu Pro Asp Phe Tyr Lys Thr Lys
               180                 185                 190
   Ile Lys Leu Cys Ile Leu Leu His Ala Phe Arg Ile Arg Ala Val Thr
           195                 200                 205
   Ile Asp Arg Val Met Ser Tyr Leu Asn Ala Ser
       210                 215
<210> 6
   <211> 328
   <212> PRT
   <213> Homo sapiens
<400> 6
   Met Cys His Gln Gln Leu Val Ile Ser Trp Phe Ser Leu Val Phe Leu
   1               5                   10                  15
   Ala Ser Pro Leu Val Ala Ile Trp Glu Leu Lys Lys Asp Val Tyr Val
               20                  25                  30
   Val Glu Leu Asp Trp Tyr Pro Asp Ala Pro Gly Glu Met Val Val Leu
           35                  40                  45
   Thr Cys Asp Thr Pro Glu Glu Asp Gly Ile Thr Trp Thr Leu Asp Gln
       50                  55                  60
   Ser Ser Glu Val Leu Gly Ser Gly Lys Thr Leu Thr Ile Gln Val Lys
   65                  70                  75                  80
   Glu Phe Gly Asp Ala Gly Gln Tyr Thr Cys His Lys Gly Gly Glu Val
                   85                  90                  95
   Leu Ser His Ser Leu Leu Leu Leu His Lys Lys Glu Asp Gly Ile Trp
               100                 105                 110
   Ser Thr Asp Ile Leu Lys Asp Gln Lys Glu Pro Lys Asn Lys Thr Phe
           115                 120                 125
   Leu Arg Cys Glu Ala Lys Asn Tyr Ser Gly Arg Phe Thr Cys Trp Trp
           130                 135                 140
   Leu Thr Thr Ile Ser Thr Asp Leu Thr Phe Ser Val Lys Ser Ser Arg
   145                 150                 155                 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
       210                 215                 220
   Ser Ser Phe Phe Ile Arg Asp Ile Ile Lys Pro Asp Pro Pro Lys Asn
   225                 230                 235                 240
   Leu Gln Leu Lys Pro Leu Lys Asn Ser Arg Gln Val Glu Val Ser Trp
                   245                 250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser
                   325
<210> 7
   <211> 144
   <212> PRT
   <213> Homo sapiens
<400> 7
   Met Trp Leu Gln Ser Leu Leu Leu Leu Gly Thr Val Ala Cys Ser Ile
   1               5                   10                  15
   Ser Ala Pro Ala Arg Ser Pro Ser Pro Ser Thr Gln Pro Trp Glu His
               20                  25                  30
   Val Asn Ala Ile Gln Glu Ala Arg Arg Leu Leu Asn Leu Ser Arg Asp
           35                  40                  45
   Thr Ala Ala Glu Met Asn Glu Thr Val Glu Val Ile Ser Glu Met Phe
       50                  55                  60
   Asp Leu Gln Glu Pro Thr Cys Leu Gln Thr Arg Leu Glu Leu Tyr Lys
   65                  70                  75                  80
   Gln Gly Leu Arg Gly Ser Leu Thr Lys Leu Lys Gly Pro Leu Thr Met
                   85                  90                  95
   Met Ala Ser His Tyr Lys Gln His Cys Pro Pro Thr Pro Glu Thr Ser
               100                 105                 110
   Cys Ala Thr Gln Ile Ile Thr Phe Glu Ser Phe Lys Glu Asn Leu Lys
           115                 120                 125
   Asp Phe Leu Leu Val Ile Pro Phe Asp Cys Trp Glu Pro Val Gln Glu
       130                 135                 140

## Description

### Field of the invention

The present invention relates to cancer therapy. In particular the invention relates to an oncolytic vaccinia virus and virus vectors for cancer therapy.

### Background to the invention

Despite advances in minimally invasive surgery, hyperfractionated radiotherapy and new combinations of chemotherapeutic agents, the survival rates for patients with many solid tumour types have remained unchanged. Oncolytic viruses are attractive therapeutics for treatment of cancers that are resistant to conventional therapies

Oncolytic viruses are viruses that can specifically target and kill cancer cells. Additionally, oncolytic viruses can also provide the immunostimulatory signals necessary to increase the host's own anticancer response.

Vaccinia virus is a double stranded DNA virus with many features which make it an attractive candidate for oncolytic therapy. It shows rapid replication, efficient spread to tumours and strong lytic ability. Additionally, vaccinia has been studied extensively and has a well-defined molecular biology with a large cloning capacity and a variety of commercially available natural and synthetic promoters making it ideal as a vector for carrying heterologous nucleic acid sequences. Vaccinia has well established safety profile and treatments for uncontrolled infections are readily available. Furthermore, a hypoxic microenvironment commonly found in solid tumours is detrimental to the replication and efficacy of many types of oncolytic viruses, but not to vaccinia virus.

Several strains of oncolytic vaccinia virus have been reported, for example the Western Reserve, Wyeth and Lister strains. Various deletion mutants of each of these strains have been created. McCart et al (Cancer Res. 2001, 61; 8751-8757) describe a version of the Western Reserve (WR) strain with deletions in the thymidine kinase (TK) gene and the viral growth factor (VGF) gene. These deletion mutants are capable of efficiently cross-priming the immune system against tumour antigens. However, biodistribution studies demonstrated significant viral titres in normal ovarian tissue and to a lesser extent in bone marrow, raising the prospect of infertility and myelosuppression following vaccinia virus treatment. Hung et al (Gene Therapy, 2007, 14; 20-29) describes a TK-deficient vaccinia Lister strain. However, this strain also showed localisation in normal ovary in addition to ovarian tumours.

Insertion of heterologous genes for example cytokine encoding genes into the virus can further drive the immune response. However, the insertion of cytokines can decrease the efficacy of viral replication through early viral clearance. This has indeed been observed in vivo with vaccinia armed with some immunomodulatory genes such as IL-2, IL-15, TNF and CD40 ligand.

Furthermore, Hiley et al. (Gene Therapy. 2010, 17; 281-287) describes Lister strain vaccinia virus as a potential therapeutic vector targeting hypoxic tumours. Dimier et al. (Journal of Virology. 2011, 85, 10; 5016-5026) describes that deletion of major nonessential genomic regions in the vaccinia virus Lister strain enhances attenuation without altering vaccine efficacy in mice. Bartlett et al. (Journal Of General Virology. 2002, 83, 8; 1965 - 1976) describes that vaccinia virus N1L protein is an intracellular homodimer that promotes virulence. Billings et al. (Ann. N.Y. Acad. Sci. 2004, 1030; 297-302) describes that lack of N1L gene expression results in a significant decrease of vaccinia virus replication in mouse brain. Postigo et al. (Journal of Virology. 2011, 86, 1; 203-213) describes that vaccinia virus encoded Bcl-2 homologues do not act as direct Bax inhibitors. CN 103 110 939 describes the sequential use of adenovirus and vaccinia virus as a vaccine for use in the treatment of cancer. Jahangir et al. (Molecular Therapy. 2014, 22, S1; S248 - S249) describes a tumour selective oncolytic vaccinia virus lacking the N1L gene that enhances the anti-tumour immune response when used as an anticancer therapeutic.

Despite the progress that has been made in the field of oncolytic viruses, no therapeutic product based on vaccinia has yet reached the market. There is therefore an unmet need for more effective oncolytic vaccinia for the treatment of cancer.

### Summary of the invention

In a first aspect, the present invention provides a TK-deficient vaccinia virus comprising an inactivated N1L gene, wherein the N1L gene is inactivated by the insertion of a nucleic acid sequence encoding a heterologous polypeptide, and wherein the nucleic acid sequence comprises at least three vaccinia virus promoters, said vaccinia virus promoters are positioned in the same orientation which is opposite to that of the intact vaccinia virus ORF L024.

The vaccinia virus promoters may be selected from the group consisting of mH5, H5, P7.5 and PE/L.

The polypeptide may be a cytokine.

The polypeptide may be selected from the group consisting of GM-CSF, IL-12 and IL-21.

In a second aspect there is provided a TK-deficient vaccinia virus according to the first aspect of the invention for use in cancer therapy.

In a third aspect there is provided a composition comprising a TK-deficient vaccinia virus according to the first aspect of the invention, optionally in the presence of a pharmaceutically acceptable carrier or excipient.

The polypeptide may be a cytokine selected from the group consisting of GM-CSF, IL-12 and IL-21.

The composition may be for use in cancer therapy.

### Detailed description of the invention

As described herein, there is provided a nucleic acid sequence comprising at least three vaccinia virus promoters wherein the at least three promoters are positioned in the same orientation in the nucleic acid sequence.

A linear DNA has two possible orientations - the 5' to 3' direction and the 3' to 5' direction. For example, if one promoter is positioned in the 5' to 3' direction, and if a second promoter is also positioned in the 5' to 3' direction within the same polynucleotide molecule/strand, then the two promoters are positioned in the same orientation.

The nucleic acid sequence may be natural, synthetic or recombinant. It may, for example, be cDNA, PCR product or a genomic sequence. It may be isolated, or as part of a plasmid, vector or host cell. A plasmid is a circular extrachromosomal DNA molecule with the ability to replicate independently of chromosomal DNA.

A plasmid may be used to introduce an expression cassette into a host cell. Plasmids may also be used to express a polypeptide in a host cell. For example a bacterial host cell may be transfected with a plasmid capable of encoding a particular polypeptide, in order to express that polypeptide. The term also includes yeast artificial chromosomes and bacterial artificial chromosomes which are capable of accommodating longer portions of DNA.

A promoter is a region of DNA with a specific sequence that initiates the transcription of a particular gene or genes. Promoters used for the expression of heterologous genes in vaccinia include promoters controlling early and late transcriptional activity, for example mH5, H5, P7.5 and PE/L. A heterologous gene, as used herein, is a gene that is not normally found in the virus. The modified H5 promoter, mH5, has a predominantly early activity and shows greater stability than the naturally occurring H5. Preferably, the at least three promoters are mH5.

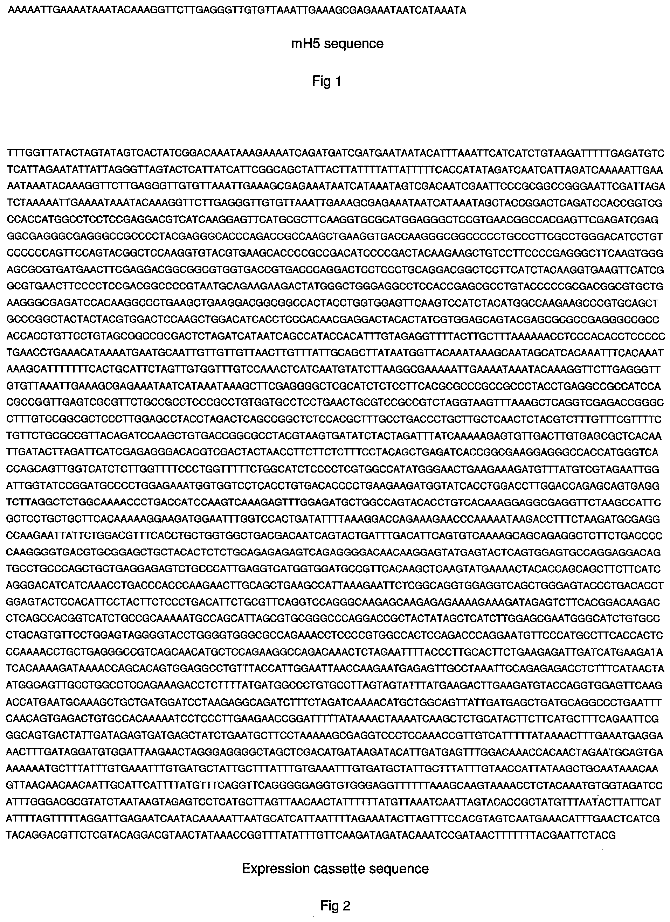

As described herein, the promoter comprises a nucleotide sequence that is substantially homologous to the sequence set forth in Fig 1. Nucleic acid sequences with greater than 20% identity (for example 25%, 30%, 40%, 50%, 60%, 70%, 80%, 90%, 95% or 99%) are considered to be homologous sequences. As used herein, substantially homologous refers to sequences exhibiting at least 60% or 70%, preferably means at least 80%, more preferably at least 90%, and most preferably at least 95%, 96%, 97%, 98%, 99% or greater identity. In an embodiment of the invention the sequence has at least 80% or more (e.g., 85%, 90%, 95%, 96%, 97%, 98%, 99%, 99.5%, etc.) homology with the sequence set forth in Fig 1.

As used herein, the terms homology and identity are interchangeable.

Sequence comparisons to determine homology can be carried out using readily available sequence comparison software. Examples include but are not limited to BLAST (see Ausubel et al., 1999 Short Protocols in Molecular Biology, 4th Ed - Chapter 18) and FASTA (Altschul et al., 1990 J. Mol. Biol. 403-410). Both BLAST and FASTA are available for offline and online searching (see Ausubel et al., 1999, Short Protocols in Molecular Biology, pages 7-58 to 7-60).

As described herein, the nucleic acid sequence is present in a vector. A vector as used herein refers to a construct for introducing a nucleic acid sequence into a cell or a virus for expression or replication. It refers to a recombinant construct for example a plasmid, a virus or any other construct capable of expression or replication of the nucleic acid sequence upon introduction into a cell or virus.

The nucleic acid sequence described herein may be part of an expression cassette. An expression cassette is a part of a vector. It comprises a promoter, an open reading frame and a 3' untranslated region.

As described herein, the vaccinia virus vector comprises a nucleotide sequence that has at least 80% or more (e.g., 85%, 90%, 95%, 96%, 97%, 98%, 99%, 99.5%, etc.) homology with the sequence set forth in Fig 2.

As described herein, the nucleic acid sequence encodes a heterologous polypeptide.

A polypeptide as used herein refers to a plurality of amino acid residues joined together by peptide bonds. It is used interchangeably with protein, peptide, oligopeptide and includes glycoproteins and derivatives thereof. The term "polypeptide" is also intended to cover analogues and derivatives of polypeptides which retain the same biological function or activity as the original polypeptide.

A heterologous polypeptide as used herein refers to any polypeptide that is not normally expressed by the virus in nature. The heterologous polypeptide can be biologically active. A biologically active polypeptide as used herein refers to a polypeptide that has a biological function or activity.

As described herein, the biologically active polypeptide is therapeutic. A therapeutic polypeptide is a polypeptide that has been or is being developed for therapeutic use. Examples of a therapeutic polypeptide include but are not limited to cytokines, chemokines and growth factors.

The cytokine may be an immunomodulating agent such as an interleukin (e.g. IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35 and IL-36), an interferon (INF-α, INF-β, INF-γ and INF-ω), tumour necrosis factor (TNF) and/or granulocyte macrophage colony-stimulating factor (GM-CSF).

As described herein, the polypeptide is an interleukin. As described herein, the polypeptide is IL-12. IL-12 may be derived from any animal e.g. human (hIL-12), mouse (mIL-12), horse, cow, pig, etc. It may be natural or recombinant. Preferably the nucleotide sequence encoding IL-12 is a full length IL-12 gene. As described herein, the nucleotide sequence encodes 30%, 40%, 50%, 60%, 70%, 75%, 80%, 85%, 90%, 95%, 97%, 98%, or 99% of a full-length gene. As described herein, the polypeptide is GM-CSF, or the polypeptide may be IL-21. All features which refer to IL-12 as described above shall apply mutatis mutandis to GM-CSF and IL-21.

References to IL-12 herein include IL-12A (for example GenBank Accession no. AF404773.1 GI:15128214) and/or IL-12B (for example GenBank Accession no. AY008847.1 GI:11192034). The mature IL-12 protein includes both subunits. References to IL-21 herein include isoform 1 (for example GenBank Accession no. NP_068575.1 GI:11141875) and/or isoform 2 (GenBank Accession no. NP_001193935.1 GI:333033767). References to GM-CSF herein include GenBank Accession no. AF373868.2 GI:14278709. Generally, the sequences are human sequences.

A heterologous polypeptide could also be a reporter polypeptide. Reporter polypeptide as used herein refers to a polypeptide whose expression is indicative of the presence of the nucleic acid sequence, expression cassette or vector in a host cell or virus. Examples of reporter polypeptides include but are not limited to fluorescent polypeptides, chemiluminescent polypeptides, bioluminescent polypeptides, phosphorescent polypeptides as well as enzymes.

As described herein, the reporter polypeptide is a fluorescent polypeptide. Fluorescent polypeptides include but are not limited to green fluorescent protein, red fluorescent protein, yellow fluorescent protein, cyan fluorescent protein and their derivatives.

Restriction sites are specific nucleotide sequences that are recognised and cleaved by restriction enzymes. Examples of restriction enzymes are SaII, BgIII, Hindlll, Smal, BamHI and Mlul. A BamHI restriction site is a restriction site recognised by BamHI. The restriction sites for other enzymes are similarly named.

As described herein, the nucleic acid sequence or vector comprises one or more restriction sites. As described herein, is a nucleic acid sequence or vector comprising SaII, BgIII, HindIII, Smal, BamHI and Mlul restriction sites.

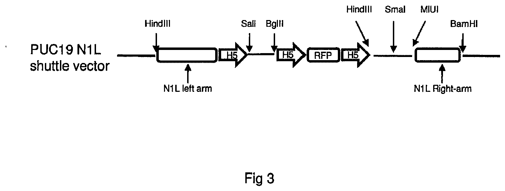

As described herein, the nucleic acid sequence or vector is comprised within a vaccinia virus. As described herein, the nucleic acid sequence has the formula shown in Fig 3.

Vaccinia has evolved a number of strategies to evade the host immune system. For example, the virus secretes a number of proteins that inhibit cytokines and chemokines which are involved in the host's antiviral response. One such protein is the N1 gene product, N1L, which is believed to inhibit apoptosis of infected cells as well as NF-kB activation. NF-kB is a transcription factor that controls the production of a number of cytokines that are involved in viral clearance. N1L gene deletion has been shown to lead to an increase in pro-inflammatory antiviral cytokines controlled by NF-kB such as IL1β, TNFα and IFNα/β. N1L has also been shown to modulate natural killer (NK) cell response. NK cells are generally the first line of host defence to viral infection. Deletion of N1L has been shown to induce an increased NK cell local activity. Consistent with these findings, Bartlett et al (J General Virology, 2002, 83:1965-1976) have shown that compared to a wild-type VV strain, an N1L-deleted Western Reserve vaccinia strain is cleared more rapidly by the host immune response. As used herein immune response means the reaction of the immune system against a foreign substance.

As described herein, there is provided a vaccinia virus comprising a nucleic acid sequence or vector as described herein, wherein the nucleic acid sequence is inserted into the N1L gene.

Insertion of a nucleic acid sequence into a target sequence can be facilitated by methods well known to the person skilled in the art. For example, methods described in Molecular Cloning, A Laboratory Manual, Second Edition, by J. Sambrook, E. F. Fritsch and T. Maniatis (2003), Cold Spring Harbor Laboratory Press, Virology Methods Manual, edited by Brian W J Mahy and Hillar O Kangro (1996) Academic Press and Expression of genes by Vaccinia virus vectors. Current Protocols in Molecular Biology, published by John Wiley and Son (1998), Chapter 16. As described herein, the nucleic acid sequence is inserted into the N1L gene by homologous recombination.

As described herein, there is provided a TK-deficient vaccinia virus comprising an inactivated N1L gene.

There are multiple strains of vaccinia with varying levels of virulence for humans and animals. A number of strains of the virus were used around the world as part of the smallpox eradication programme in the 1950s. Different strains were used in different areas of the world, for example, the New York City Board of Health (NYCBOH) strain and its derivative, Wyeth, were popular in the United States, whereas Copenhagen (CPN) and Lister strains were predominant in Europe. As described herein, the vaccinia strain is Lister.

A TK-deficient vaccinia virus as used herein refers to a vaccinia virus that shows a phenotype consistent with a lack of endogenous thymidine kinase (TK). A TK-deficient vaccinia virus is dependent on thymidine kinase produced by the host cell. Thymidine kinase is constitutively produced in tumour cells but not in normal cells. A TK-deficient vaccinia virus can therefore survive selectively in tumour cells, especially with activation of EGFR/Ras/ERK pathways. A host cell is any cell that the virus can infect.

As described herein, the TK-deficient vaccinia virus comprises an inactivated N1L gene. Inactivation as described herein refers to silencing of the gene at the transcriptional or post transcriptional level, deletion of the gene, mutation in the gene, disruption of the gene by insertion of a nucleic acid sequence or any other method that renders the virus unable to create a fully functional gene product. Inactivation of a gene can be partial or complete. As described herein, the inactivation of N1L is by insertion of a nucleic acid sequence. The insertion can be facilitated by homologous recombination.

The inserted nucleic acid sequence may be comprised in a vector or an expression cassette.

As described herein, the nucleic acid sequence encodes a heterologous polypeptide. The heterologous polypeptide could be biologically active. As described herein, the biologically active polypeptide is therapeutic.

As described herein, the nucleic acid sequence encodes an RNAi-inducing agent, RNAi agent, siRNA, shRNA, miRNA, antisense RNA, ribozymes, catalytic DNA and the like. As described herein, the nucleic acid sequence encodes a radiation and/or chemotherapy sensitiser.

As described herein, there is provided a composition comprising a TK-deficient vaccinia virus. As described herein, the composition optionally comprises a pharmaceutically acceptable carrier, diluent or excipient.

The composition may be adapted for administration by any appropriate route, for example by the oral (including buccal or sublingual), topical (including buccal, sublingual or transdermal), or parenteral (including subcutaneous, intramuscular, intravenous, intra-arterial, intra-thecal, intra-pleural, intra-ophthalmological, intra-cardiac, intraperitoneal or intradermal) route.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents.

Excipients which may be used for injectable solutions include water, alcohols, polyols, glycerine and vegetable oils, for example. The compositions may be presented in unit-dose or multidose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

As described herein, there is provided a method of treating cancer comprising administrating to a subject a therapeutically effective amount of a TK-deficient vaccinia virus comprising a vector or nucleic acid sequence encoding a heterologous polypeptide wherein said virus further comprises an inactivated N1L gene.

As used herein, a subject refers to an animal, including a human being. An animal can include mice, rats, fowls such as chicken, ruminants such as cows, goat, deer, sheep and other animals such as pigs, cats, dogs and primates such as humans, chimpanzees, gorillas and monkeys.

A therapeutically effective amount is the dose sufficient to induce oncolysis. Doses for delivery and administration can be based upon current existing protocols, empirically determined, using animal disease models or optionally in human clinical trials. Initial study doses can be based upon animal studies set forth herein, for a mouse, for example. Doses can vary and depend upon whether the treatment is prophylactic or therapeutic, the type, onset, progression, severity, frequency, duration, or probability of the disease to which treatment is directed, the clinical endpoint desired, previous or simultaneous treatments, the general health, age, gender, race or immunological competency of the subject and other factors that will be appreciated by the skilled artisan. The dose amount, number, frequency or duration may be proportionally increased or reduced, as indicated by any adverse side effects, complications or other risk factors of the treatment or therapy and the status of the subject. The skilled artisan will appreciate the factors that may influence the dosage and timing required to provide an amount sufficient for providing a therapeutic or prophylactic benefit.

As described herein, the method further comprises administering to the subject an additional cancer therapy. Cancer therapy as used herein refers to refers to treatment of cancer by any medical or physical means. The additional cancer therapy can be chemotherapy, biological therapy, radiotherapy, immunotherapy, hormone therapy, anti-vascular therapy, cryotherapy, toxin therapy and/or surgery, including combinations thereof.

Methods and uses of the invention as disclosed herein can be practiced immediately or days, months or years after a subject has been identified as having the disease targeted for treatment.

The methods include administering the virus at different schedules. A single dose of the virus may be administered to a subject or a tumour over a 1, 2, 5, 10, 15, 20, or 24 hour period. The virus may be administered over 1, 2, 3, 4, 5, 6, 7 or more days or weeks. The interval between injections can be 1, 2, 3, 4, 5, 6, 7 days or weeks. Typically, multiple doses are administered to the same general target region, such as in the proximity of a tumour or in the case of intravenous administration a particular entry point in the blood stream or lymphatic system of a subject. The vaccinia virus vector may be administered 2, 3, 4, 5, or more times. The vaccinia virus vector could be given before resection of tumours at different schedule and doses.

The methods include administering the virus at different viral concentrations. In certain aspects, the subject is administered at least 5x10⁷, 1x10⁸, 2 x 10⁸, 5 x 10⁸, 1 x 10⁹, 2 x 10⁹, 5 x 10⁹, 1 x 10¹⁰, 5 x 10¹⁰, 1 x 10¹¹, 5 x 10¹¹, 1 x 10¹² or more viral particles or plaque forming units (pfu), including the various values and ranges there between. The viral dose can be administered in 0.1mL, 1mL, 2 mL, 3 mL, 4 mL, 5 mL, 6 mL, 7 mL, 8 mL, 9 mL, 10 mL or more, including all values and ranges there between. The dose may be spread over time or by separate injection.

As described herein, the subject is a human with cancer and/or a tumour. The cancer may be a gastrointestinal cancer, a respiratory tract cancer, a genitourinary tract cancer, a hematopoietic cancer, a sarcoma, an adenocarcinoma, a squamous cell carcinoma or a non-malignant tumor/hyperplasia. The tumour may be non-resectable prior to treatment and resectable after treatment. The tumour can be a recurrent, primary, metastatic, and/or multi-drug resistant tumour. In certain aspects the tumor is located on or in the pancreas. In other aspects, the tumour can be a neuroendocrine tumour, an endocrine tumour, a peripheral central nervous system tumour, a brain cancer tumor, a head and neck cancer tumor, an esophageal cancer tumour, a skin cancer tumor, a lung cancer tumor, a liver tumour, a thymic tumor, a stomach cancer tumor, a colon cancer tumour, an ovarian cancer tumor, a uterine cancer tumor, a bladder cancer tumor, a testicular cancer tumour, a bladder tumour, a rectal cancer tumour, melanoma or a breast cancer tumour.

The compositions and methods disclosed may be used in different types of gene therapy for example tumor suppressor gene therapy, suicide gene therapy, viral vector immunisation strategies, anti-angiogenic therapy, pro-apoptosis gene therapy and gene replacement therapy. "Oncolytic Viruses for Cancer Therapy: Overcoming the Obstacles" (Wong et al. Viruses 2010, 2, 78-106).

The compositions and methods disclosed may be used in combination with additional therapeutic means or methods in the treatment of cancer, for example surgery, chemotherapy, radiation therapy, molecular cancer therapy or a further gene therapy, which may be used for administering genes that are different from the herein described nucleic acids of the invention.

Preferred aspects of the second and subsequent aspects of the invention are as for the first aspect *mutatis mutandis*

Cell lines: All tumour cell lines used were stored in the inventors' lab, either from ATCC or Cancer Research UK Cell line Service Unit or kindly provided by collaborators. All human cancer cell lines were genotyped by STR assay. The murine tumour cell lines used in this study include: the colorectal cancer cell line CT26 was derived from the BALB/c strain, whilst CMT93 (colorectal), LLC (Lewis lung cancer) and B16-F10 (metastatic melanoma) originated from the C57BL/6 strain. SCC7 is a head and neck cancer-derived squamous carcinoma, from C3H/HeN mice and was kindly donated by Dr Osam Mazda (Department of Microbiology, Kyoto Prefectural University of Medicine, Japan), MOSEC is a murine ovarian carcinoma cell line. Panc02 is a chemically induced murine pancreatic carcinoma cell line; DT6606 (pancreatic carcinoma) originated from a C57BL/6 strain transgenic mouse with mutation in the K-Ras conditional to the pancreas. This was a kind gift of Professor David Tuveson (CRUK, Cambridge Research Institute, Cambridge, UK). In addition, our group had previously stably transfected the DT6606 cell line with a plasmid containing the chicken ovalbumin gene (DT6606-ova). Finally, CV1 is an African Green Monkey "normal" kidney cell line obtained from the ATCC, VA, USA and was used as a stock cell line to facilitate the mass production of viruses as well as in all viral titration assays. The human cancer cell lines used in this invention include: the human pancreatic carcinoma cell lines SUIT-2, MIAPaCa2, PANC1, PT-45 and Capan-2; the human colorectal carcinoma cell lines HT29, HCT116, and SW620, gastric adenocarcinoma MKN45 and the human ovarian carcinoma cell line A2780.

Viruses: VVL15 was constructed by the insertion of the lacZ reporter and the firefly luciferase genes into the TK region of the Lister strain of vaccinia virus under the control of the synthetic early/late and p7.5 promoters respectively using an in vitro intracellular recombination technique previously described in Timiryasova TM et al. Biotechniques. 2001; 31:534, 6, 8-40. VVL15 is TK-deficient.

WRLuc, TK deletion, and WRDD, double deletion (TK and VGF) viruses, were kindly provided by Dr Steve Thorne (University of Pittsburgh, USA) and Dr A. McCart (University of Toronto, Canada) respectively.

Mass viral production: The primary viral expansion from above was rapidly freeze-thawed twice and diluted into the necessary volume of 5% FCS CM required to infect between 36-40 T175 flasks containing CV1 cells (at 80-90% confluence). 48 hr later, infected CV1 cells were harvested and through repeated rounds of centrifugation at a speed of 2,000 rpm for 5 minutes (at 4°C), collected into a single pellet. The pellet was washed in PBS, re-suspended in 12ml of 10 mM Tris-HCI (pH 9) buffer and stored at -80°C for purification at a later date.

Viral purification: The concentrated viral lysate suspension from above was freeze-thawed twice and transferred to a dounce homogeniser (Thermofisher) and homogenised via 60 strokes. It was then subjected to ultrasonication for 30 seconds. Following centrifugation at 2,000 rpm at 4°C for 5 minutes, the supernatant (containing released virion particles) was collected and diluted to a total volume of 30ml with 10mM Tris-HCI buffer. The solution was divided into four aliquots; each layered gently onto 17 ml of a 36% glucose solution in a 36ml Beckman ultracentrifuge tube and centrifuged at 13,500 rpm for 80 minutes at 4°C. The resulting pellets were re-suspended to a total of 16ml in 10 mM Tris-HCI, divided into four again and carefully layered onto another four glucose gradients, this time graded from 25% w/m near the surface to 40% at the base of each tube. A second round of ultracentrifugation was performed. This was necessary to remove further particulate cellular debris, which could be toxic when administered intravenously into mice. The final pellets were re-suspended in 1-4ml of viral re-suspension buffer (PBS; 10% glycerol; 138mM NaCl; pH 7.4). A sample of purified virus was titrated via a TCID50 assay as described below.

Viral replication: Cells were seeded at 2 to 4 × 10⁵ cells per well, depending on growth rates, in three wells of 6-well plates in medium with 10% FCS, and infected with 1 PFU/cell of vaccinia viruses 16-18 hours later. Samples were harvested in triplicate at 24-hour intervals up to 72 hours. Viral replication was detected by TCID50 (50% tissue culture infective dose) as described in Wang et al (J Clin Invest, 2009, 119:1604-1615).

Statistical Analysis: Unless otherwise mentioned, Graphpad Prism 5 was used for comparative statistical analysis. Dual condition comparisons were made using the unpaired student t test. For more than one condition or for an additional variable such as time, a 1 or 2-way ANOVA respectively was performed. Post hoc tests (Knewman-Keuls for one-way ANOVA and Bonferroni for 2-way ANOVA) compared specific pairs of conditions within the experiment. Survival data were represented as a Kaplan-Meier plot with log rank analysis to delineate whether any differences between groups were statistically significant.

The invention will now be further described by way of reference to the following Examples which are present for the purposes of reference only and are not to be construed as being limiting on the invention.

Reference is made to a number of drawings in which:
FIGURE 1 shows the sequence of the modified vaccinia promoter mH5.
FIGURE 2 shows the sequence of the expression cassette comprising three mH5 promoters.
FIGURE 3 shows the formula of the vector comprising a nucleic acid sequence according to the invention.
FIGURE 4 shows a schematic representation of VVL15N1L vectors and the N1L pShuttle plasmid used to create new VVL15N1L vectors. The vectors are named as indicted. The long horizontal bar depicts the double stranded DNA genome of VV. L024, N1L (L025), L026 and TK refer to transcription units.
FIGURE 5 shows the confirmation of N1L deletion in VVL15N1L vectors.
FIGURE 6 shows the biological distribution of VVL15 and VVL15N1L vectors in tumour tissue (Fig 6a) and off-site locations (Fig 6b and 6c).
FIGURE 7 shows the replication of VVL15N1L in different cell lines. The graphs on the left represent replication curves of VVL15 (solid) in comparison to VVL15-N1L (dashed); whereas those on the right correspond to VVL15-RFP (solid) versus VVL15-N1L (dashed).
FIGURE 8 shows the cytotoxic potency of VVL15N1L (hatched) compared with VVL15-RFP (solid) different cell lines.
FIGURE 9 shows the cytotoxic potency of VVL15N1L and VVL15N1L armed with mIL-12 or mGM-CSF.
FIGURE 10 shows IFN-γ production in splenocytes co-cultured with growth-arrested SCC7 cells (Fig 10a) and heat-inactivated VVL15 (Fig 10b) and treated with VVL15, VVL15N1L or PBS.
FIGURE 11 shows IFN-γ production in splenocytes co-cultured with DT6606-ova cells (Fig 11a), ovalbumin antigen (Fig 11b) and B8R peptide (Fig 11c) and treated with VVL15, VVL15N1L or PBS.
FIGURE 12 shows IFN-γ production in splenocytes co-cultured with growth-arrested LLC cells (Fig 12a) and B8R peptide (Fig 12b) following treatment with VVL15, VVL15N1L or PBS.
FIGURE 13 shows the efficacy of VVL15N1L in pancreatic cancer mouse model - tumour growth rate in DT6606 (Fig 13a) and CMT-93 (Fig 13c) following treatment with VVL15, VVL15N1L or PBS and survival rate in DT6606 (Fig 13b) and CMT-93 (Fig 13d) flank tumour models following treatment with VVL15, VVL15N1L or PBS.
FIGURE 14 shows the efficacy of VVL15N1L in orthotopic lung cancer mouse model. Fig 14a demonstrates the individual weight profiles of PBS-treated mice, Fig 14b demonstrates the mean weight of mice in each group as a function of time, Fig 14c and 14d, the corresponding Kaplan-Meier survival curves and median survival plots respectively.
FIGURE 15 shows tumour volumes in LLC tumour model following IT administration of VVL15RFP, VVL15N1L or PBS (Fig 15a) and metastases in each treatment group at sacrifice (Fig 15b).
FIGURE 16 shows tumour growth in DT6606 flank models following treatment with VVL15N1L, VVL15N1L-mIL-12, VVL15N1L-mGM-CSF or PBS (Fig 16a) and the corresponding Kaplan Meir survival curves (Fig 16b).
FIGURE 17 shows the assessment of IL-21 armed VV *in vitro.*
FIGURE 18 shows the anti-tumour efficacy of VV-ΔTkΔN1L-mIL-21, VV-ΔTkΔN1L-hIL-21 and control virus VV-ΔTkΔN1L.

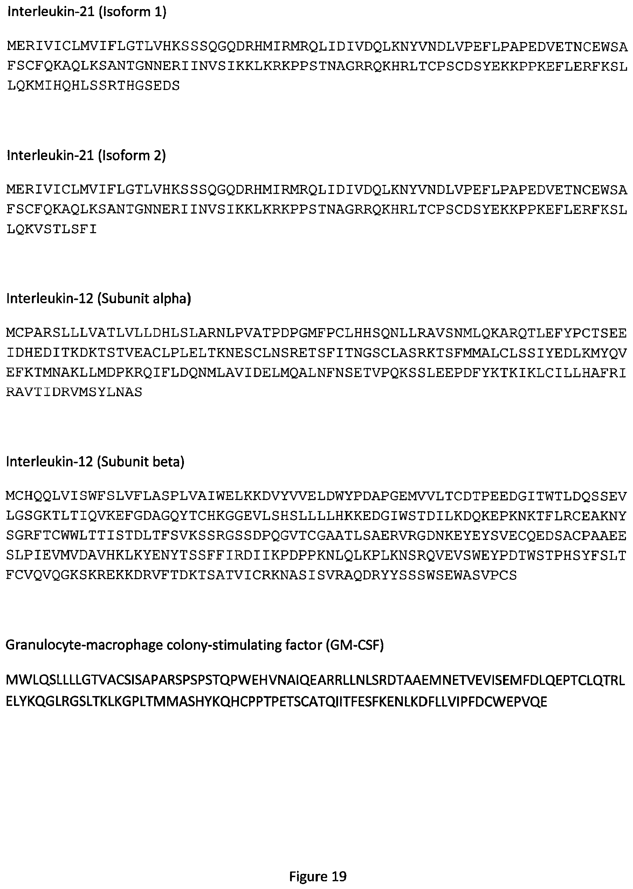

FIGURE 19 shows sequences of human IL-12A, IL-12B, IL-21 isoform 1, IL-21 isoform 2 and GM-CSF.

### Examples

### Example 1: Construction of pUC19-N1L shuttle vector

The inventors constructed a pUC19-N1L shuttle vector illustrated in Fig 3 comprising a specific expression cassette flanked by a fragment containing L024 as well as 31bp of L025 (left arm), and a fragment containing 22bp of L025 as well as L026 (right arm). The expression cassette has the following features: (1) there are three vaccinia virus mH5 promoters, and under each promoter there is a cloning restriction enzyme site for easy insertion of any gene of interest; (2) a reporter gene RFP is driven by one mH5 promoter for positive selection of recombination virus; (3) mH5 promoter only drives the expression of the inserted gene from left to right. The homologous recombination strategy used in this invention was designed to replace almost the entirety of the coding sequence of the L025 (N1L) locus. Sequence analysis at the junctions of L024/25 and L025/26 confirmed that the ORFs upstream (22bp) and downstream (31bp) of L025 remained intact.

The cDNA of m-GM-CSF, h-GM-CSF, m-IL12 and h-IL12 were cloned using standard techniques into the vector for expression under control of the mH5 promoter using the appropriate restriction enzyme to synthesise pUC19 super shuttle vectors.

### Example 2: Construction of VVL15N1L

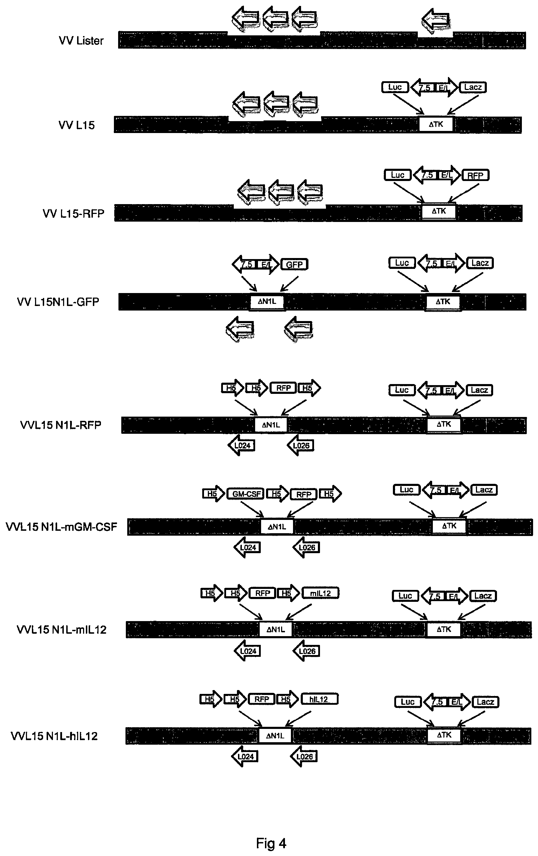

Figure 4 depicts vaccinia virus Lister strain and various vaccinia virus constructs created by the inventors. Each pUC19 super-shuttle vector was transfected (using an Effectene-based protocol - Qiagen) into CV1 cells that had been pre-infected (2 hr earlier) with VVL15 (0.1 PFU per cell). 48 hr later, the presence of red fluorescence under fluorescence microscope confirmed expression of the relevant cassette, either from the cytoplasmic plasmid or from the relatively few viruses in which homologous recombination had been successful. These latter were selected out as follows. Cells and supernatant were harvested by scraping the cells from the dish and freeze-thawing twice. 1µl of this lysate was used to infect all 6 wells of a six-well plate containing CV1 cells grown to 80-90% confluence. This low viral load would ensure the emergence of well separated plaques.

After a further 48 hr, each well was carefully scrutinized under fluorescence microscope searching for those virus-induced plaques that fluoresced red. Upon identification of positive colonies, their location was marked on the under surface of the plate with a fine-tipped permanent marker. The colony was carefully picked with a 20 µl tip filled with 5 ul 5% FCS CM after aspirating the medium from the well. The tip was then submerged into a cryotube containing 250 µl of 5% FCS CM. Following further freeze-thaw cycles, 5-20 µl of this virus solution was added to each well of a new 6-well plate containing CV1 cells as before. This process was repeated until every plaque fluoresced red, i.e., all viral colonies were due to recombinant virus. In general, it took between 4-8 rounds of plaque purification to obtain a pure batch of recombinant virus. At this point, the viral lysate was scrape-harvested and viral DNA was extracted via a column-based system (i.e., the Blood Mini Kit from Qiagen). A sample of supernatant was also tested for the presence of the relevant cytokine by ELISA. The purity of virus was confirmed by PCR amplification of the N1L gene from extracted viral DNA. Its presence would indicate contamination with the parental virus, VVL15.

Once preliminary investigations had confirmed the likely creation of a pure recombinant virus that expressed the relevant transgene, 50µl of viral lysate was added to a T175 flask containing CV1 cells, again grown to 80-90% confluence in approximately 30 ml of 5% FCS CM. Cells and media were scrape-harvested 48 hr later and kept as a "primary viral expansion".

### Example 3: Confirmation of deletion of N1L in recombinant VVLs:

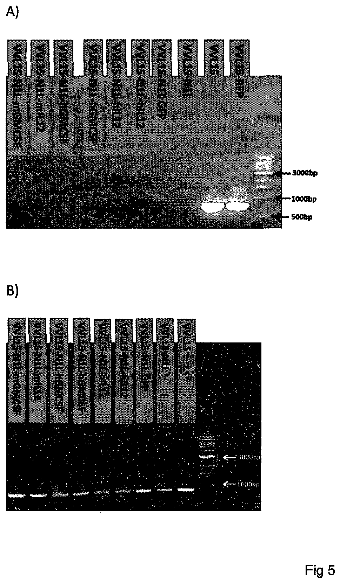

The N1L gene was deleted in all novel VVL recombinants (Fig 5a). Sense and anti-sense N1L gene primers were used to amplify via PCR viral DNA that had been extracted from infected CV1 cells. Only the VVL15 viruses contained this gene. The N1L gene containing segment spanning the primer pair was expected to measure approximately 750 bp. The A52R gene was present in all VVL recombinants. Sense and anti-sense A52R gene primers were used to PCR amplify this locus from DNA extracted from infected CV1 cells. The A52R gene segment spanning the primer pair was expected to measure approximately 880 bp (Fig 5b).

### Example 4: Validation of transgene expression from N1L gene-deleted recombinant VVLs:

A sample of supernatant from the final plaque purification round of each transgene-armed recombinant virus was analysed using the relevant cytokine-specific ELISA kit, according to the manufacturer's protocol (ebioscience, Biolegend). To assess whether each cytokine transgene was expressed by the relevant recombinant virus upon tumour cell infection, the same experimental set up as described in the viral replication assay above was conducted. At 24, 48 and 72 hr after viral infection, supernatant was collected from each duplicate set of wells and the concentration of cytokine was determined by ELISA according to the manufacturer's instructions. The control samples were supernatants collected from VVL15-N1L infected wells.

### Example 5: Assessment of viral biological distribution of VVL15N1L vectors

2x10⁶ CT26 cells in 100 µl of serum free DMEM was injected subcutaneously into the shaved right flanks of 7-week old female BALB/c mice. When tumours were approximately 100 mm³ they were randomized into two groups. An IV dose of 1x10⁸ PFU of either VVL15 or VVL15-N1L was injected via the tail vein. At days 1, 3, 7 and 10 post virus injections, 3 mice from each group were sacrificed via CO₂ inhalation. Blood was collected via cardiac puncture into pre-heparinized 1.5ml Eppendorf tubes. These along with harvested tumour, brain, lungs, liver, spleen, kidneys and ovaries were immediately snap-frozen on a Petri dish floating on precooled (to -80°C) isopentane. At a later date they were thawed, weighed and homogenised (or vortexed in the case of blood) in serum-free DMEM. Samples were diluted with a volume of 5 µl per mg (or 5 µl per µl of blood). Following a further freeze-thaw cycle, tissue homogenates were subsequently titrated for live viral PFUs using the TCID50 assay.

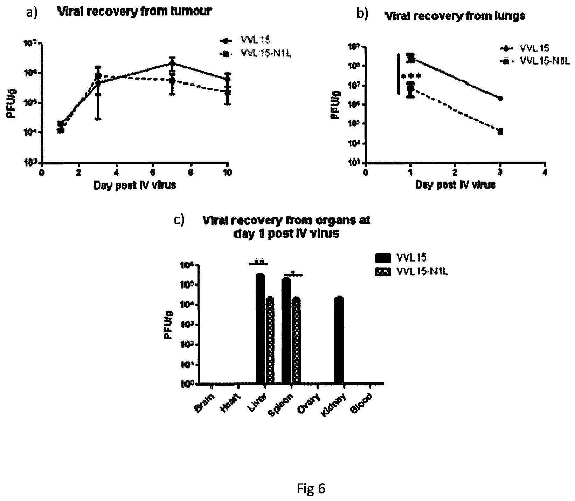

A biological distribution experiment was performed to establish whether IV-delivered virus disseminated to tumour and to determine any off-target replication (i.e. to determine the extent of tumour selectivity and thus safety). The CT26 subcutaneous flank model was utilized for this section as described in the Methods section. Following tail vein injection, both viruses could be recovered from tumour tissue until at least 10 days after injection. The peak titre is between 3 and 7 days. Unexpectedly, the viral recovery of VVL15N1L was not reduced in tumour tissues compared to VVL15 (Fig 6a).

Regarding off-target replication, with the exception of lung tissue, virus was not recovered from any other organs or blood within 24 hr post-injection. After 24 hr, VVL15-N1L viral recovery was significantly less than VVL15 from liver and spleen tissue and completely absent from kidney tissue. Neither virus was recovered at detectable levels from brain, heart, ovaries or the circulation at any time point in this experiment (Fig 6b). In contrast, virus persisted in the lungs until at least 3 days. Even in this organ, recovery of VVL15-N1L was significantly less in comparison to VVL15. The novel backbone VVL15N1L therefore appeared to have an even greater selectivity for tumour tissue than VVL15.

### Example 6: Replication of VVL15N1L in different cell lines

There are multiple mechanisms by which a tumour cell may be killed by vaccinia virus. These include innate host defences triggering apoptosis, death from virus-mediated cellular burst and host immunological defence mechanisms. If a virus is excessively cytotoxic to a cell, it may not generate enough progeny to self-propagate throughout a tumour. Furthermore its ability to replicate might be expected to correlate positively with expression of its therapeutic transgene since there will be more copies of the virus present.

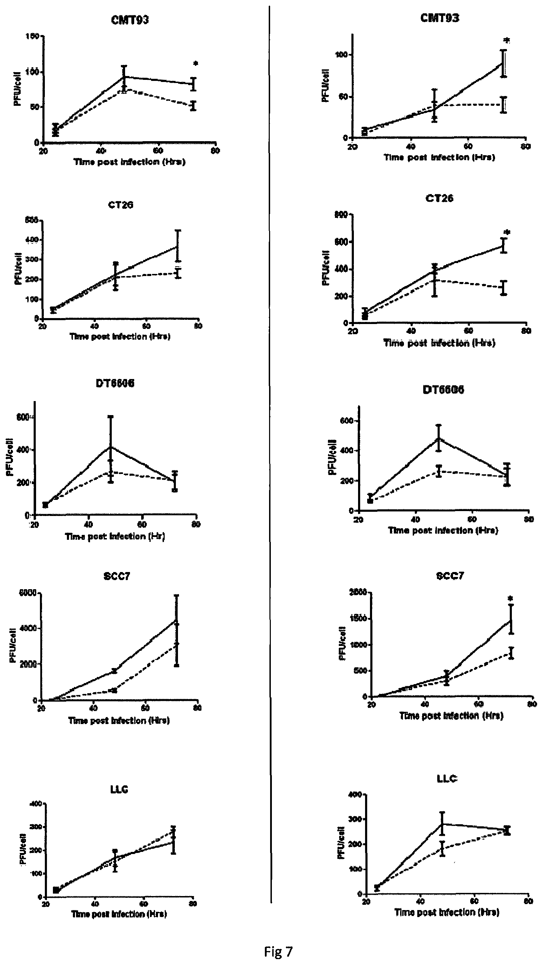

All cell lines were permissive to infection with VVL15 and VVL15N1L. The most permissive tumour cell line was SCC7, resulting in viral titres of over 1,000 PFU/per cell just three days after initially being infected with 1 PFU/per cell. This result contrasts to that from the cell cytotoxicity assay (MTS), in which SCC7 was the most resistant cell line. The cell line least permissive to replication was CMT93. Viral titres plateaued by day 3 in CMT93 and DT6606 cell lines. This is likely to reflect the out-performance of viral replication in comparison to the replication of uninfected cells (with virions effectively running out of cells to infect); coupled with possible viral degradation from proteases released from lysed cells. Statistically significant attenuation of replication of VVL15N1L was seen only in CMT-93 (Fig 7).

### Example 7: Cytotoxic potency of VVL15N1L

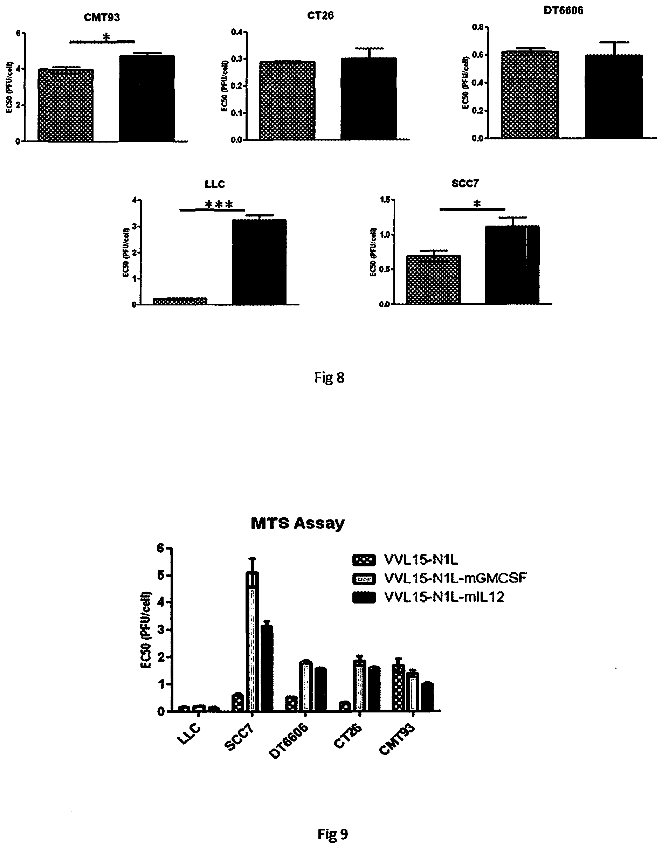

VVL15-N1L was compared with VVL15-RFP for its cytotoxicity in a range of murine cancer cell lines in vitro. Cells were seeded at 1x10³ or 1x10⁴ cells per well, depending on growth rates, in 96-well plates, and infected with viruses 16-18 hours later. Cell survival on day 6 after viral infection was determined by MTS assay and EC50 value (viral dose killing 50% of tumour cells) was calculated as described in Wang et al (J Clin Invest, 2009, 119:1604-1615). All assays were performed at least three times. Based on the EC50 values, (i.e. the PFU required to kill 50% of cells), there was no significant difference in cytotoxicity between the two viruses in CT26 and DT6606 cells. In contrast, VVL15-N1L was significantly more potent compared to VVL15 at killing CMT93, LLC and SCC7 cells (Fig 8).

The cytotoxic potency of armed VVL15N1L was also compared. EC50 values were significantly higher than VVL15-N1L (i.e., they were less potent than VVL15-N1L at killing the relevant cell line) in all cell lines except LLC and CMT93. The VVL15-N1L-mIL12 recombinant appeared to be more potent than VVL15-N1L-mGMCSF in all cell lines, a feature that reached statistical significance in SCC7 and DT6606 cells (Fig 9).

### Example 8: VVL15N1L induces a higher level of host immune response against tumour antigen

Three syngeneic in vivo tumour models were used: SCC7 cells in C3H/HeN strain mice; DT6606-ova cells in C57BL/6 strain mice and LLC cells in C57BL/6 strain mice.

Subcutaneous flank tumour models were established and treated with a single dose of virus or PBS as outlined in Table 2. To ensure timely generation of viral- and tumour-specific T cells, spleens were harvested at 14 days post infection. IFN-γ release assays were performed on the subsequently generated splenocyte suspensions.

### (1) Preparation of a single cell suspension of splenocytes from harvested spleens

Syngeneic subcutaneous flank tumours were established for the relevant tumour cell line as described below (see also Table 2). When tumours were approximately 100 mm³ in volume, mice were randomized into three groups. 1x10⁸ PFU of either VVL15 or VVL15-N1L virus in 50µl of PBS was injected intratumorally (IT) using a 1 ml insulin syringe attached to a 29-gauge needle. The needle was passed a number of times in different directions throughout the tumour during virus deployment in order for broad dissemination. The third group was injected with the equivalent volume of vehicle buffer, i.e. 50µl of PBS. 14 days after infection, animals were euthanized via CO2 inhalation. Spleens were harvested under sterile conditions, mashed through 70µm cell strainers (Becton Dickinson Falcon) using the flat end of the plunger of a 2 ml syringe and flushed through with T cell culture media (TCM) (RPMI-1640, 10% FCS, 1% streptomycin/ penicillin, 1% sodium pyruvate) into 50 ml conical flasks. Pelleted splenocytes were re-suspended in 5 ml of RBC lysis buffer (Sigma-Aldrich) following centrifugation at 1,200 rpm and left on ice for 5 minutes. After a wash-centrifugation cycle, they were re-suspended with TCM to a final concentration of 5x10⁶ cells/ml.

### (2) Preparation of growth-arrested whole tumour stimulator cells:

A single cell suspension of 5x10⁶/ ml of stimulator cells (i.e., the relevant target or control tumour cell line-SCC7, LLC or DT6606-ova) in cell culture medium (CM) was prepared in a 50 ml conical flask. A 1 mg/ml solution of Mitomycin C (MMC) (Roche) was added to this suspension to achieve a final concentration of 100µg/ml and incubated in a humidified incubator at 37°C in air with 5% CO₂ for 1 hr. The cells were subsequently washed twice with 40ml of PBS, re-suspended in 40 ml CM and incubated until ready to seed (within 30-60 minutes). The now growth-arrested stimulator cells were re-suspended in TCM to achieve a final concentration of 5x10⁵ cells/ml.

### (3) The IFN-γ release assay as a surrogate marker for tumour/antigen-specific T cell activation:

This assay is based on the release of IFNγ when memory T cells are activated by their cognate epitope-MHC complex. The splenocyte pool should contain all the cellular types (e.g. APCs, Th cells) necessary for the stimulation of CD8+ T cells. 100µl of each of the splenocyte suspensions from (1) were co-cultured with 100 µl of the target-tumour stimulator cell suspension from above in triplicate wells of a round-bottomed 96-well plate (i.e. 5x10⁵splenocytes with 5x10⁴ growth arrested tumour cells). Splenocyte-only control wells contained 5x10⁵ splenocytes in 200µl TCM. Where appropriate, splenocytes were also co-cultured with 100µl of ova-peptide (H-2Kb/ SIINFEKL, Proimmune) in TCM (to achieve a final concentration of 5µg/ml) or 100µl of TCM containing 5x10⁴ MHC-compatible, growth-arrested control tumour cells (B16-F10 when a C56BL/6 mouse-derived tumour model was used).

Furthermore, in order to prove that virus had been administered and importantly that the animal was able to mount an immune response per se, splenocytes were additionally co-cultured as above with either heat-inactivated VVL15 (100 PFU per cell, heated to 56°C for 2 hr) or a VV B8R peptide (H-2Kb/ TSYKFESV, Prolmmune), a strongly antigenic Vaccinia viral epitope (to achieve a final concentration of 5µg/ml). This experiment would also serve as a positive control for the assay itself. Plates were incubated at 37°C in air and 5% CO₂ for three days, after which they were centrifuged at 1,200 rpm for 5 minutes. The concentration of IFN-γ in supernatants taken from each of the wells was established using a murine-specific IFN-γ ELISA kit (Biolegend). The final concentration of IFN-γ, averaged across duplicate wells was determined after deduction of corresponding values obtained from wells containing splenocytes alone.

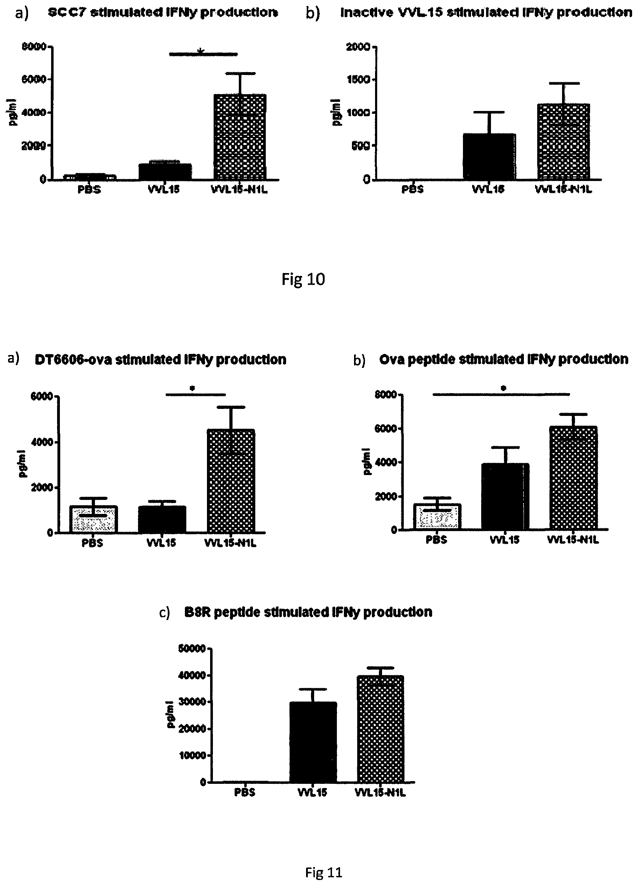

The SCC model is an aggressive murine head and neck squamous cancer model that like its counterpart in human head and neck cancers is poorly immunogenic. As demonstrated in Fig 10a-b, splenocytes from the VVL15-N1L-treated group produced significantly higher levels of IFN-γ than the VVL15 group in response to co-culture with growth-arrested SCC7 cells. There was a low but significant level of IFN-γ production from PBS-treated splenocytes. This likely reflected the host's natural immune response against tumour. As expected, there was no IFN-γ production by splenocytes in response to co-culture with heat-inactivated VVL15 in the PBS group. Surprisingly there was no significant difference between the other two groups in IFN-γ levels upon co-culture with inactivated virus (Fig 10b). It should be noted that absolute levels of IFN-γ following co-culture with inactivated VVL15 were lower in magnitude in comparison to that following co-culture with growth-arrested tumour cells. This likely resulted from variations in presented immunogenic tumour or viral epitopes. In subsequent experiments, to ensure epitope standardization, an immunogenic VV B8R epitope was used in place of inactivated VVL15.

Host immunity induced by VV in a pancreatic cancer model was also investigated. As the tumour associated antigen (TAA) profile of the DT6606 cell line had not been defined, the cell line DT6606-ova which stably expressed the foreign antigen ovalbumin was created to demonstrate the putative generation of an antigen-specific immune response (in this case an anti-ovalbumin response). This cell line was used to create a syngeneic subcutaneous flank model as described in Table 2. At 14 days post-IT injection of virus, the VVL-N1L-treated group demonstrated a significantly higher IFN-γ response from harvested splenocytes compared to the VVL15 or PBS treatment groups upon co-culture with growth-arrested DT6606-ova cells (Fig11a). There was a relatively high level of IFN-γ production in the PBS group, indeed no different from the VVL15 treatment group. Ovalbumin is a foreign antigen, with the propensity to stimulate a robust anti-ova response, so this result is unsurprising. The N1L treatment group also produced the highest level of IFN-γ when splenocytes were co-cultured with the ovalbumin antigen (although this did not reach statistical significance) (Fig 11b).

Again, the splenocyte IFN-γ response between viral groups was not statistically different following co-culture with the B8R epitope, although the magnitude of the response was nearly 10-fold higher in comparison with tumour/tumour antigen co-culture assays.

Foreign selection markers such as RFP are likely to be immunogenic and could arguably have caused the in vivo results thus far obtained. To control for this possibility, VVL15-RFP was used as the control virus (instead of VVL15) in a subcutaneous syngeneic LLC flank model (see Table 2). The experimental set up was again otherwise identical to those in SSCVII and DT6606 experiments.

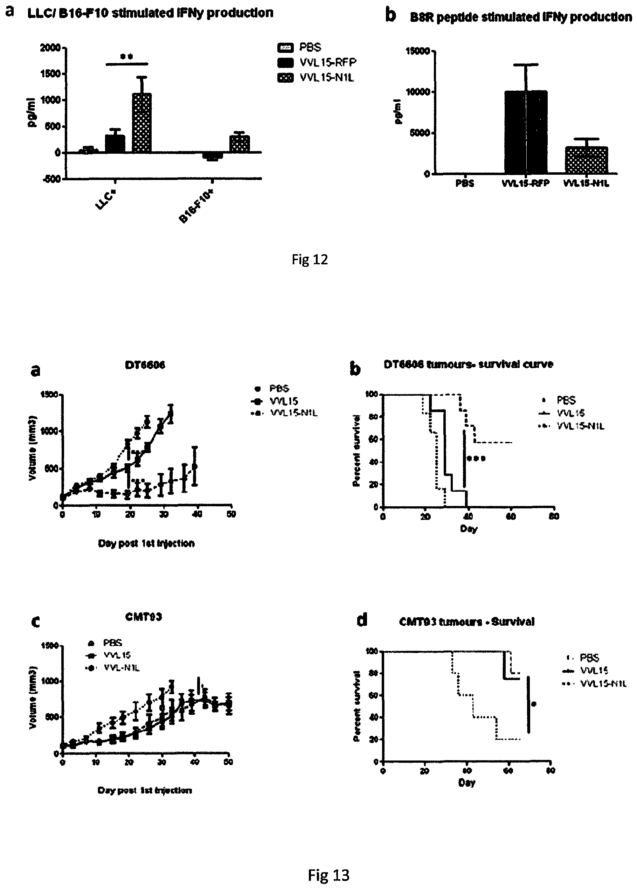

The previous results were replicated in this model, with the highest IFN-γ production demonstrated by splenocytes from the VVL15-N1L treatment arm co-cultured with growth-arrested LLC cells (Fig 12a). This was statistically significant compared to VVL15-RFP and PBS arms. There was no significant difference between viral treatment arms upon splenocyte co-culture with B8R peptide (Fig 12b). For the VVL15-N1L group, co-culture of splenocytes with growth-arrested B16-F10 cells (as an MHC haplotype-specific control stimulator cell population) led to a lower but statistically non-significant IFNγ level in comparison to co-culture with growth-arrested LLC cells (p=0.0594). It is likely that a number of tumour epitopes are shared between these and other solid tumour cell lines. CTLs generated against these could have been responsible for the IFN-γ levels obtained in the B16-F10 group.

### Example 9: Efficacy of VVL15N1L in pancreatic cancer model

Either 5x10⁶ CMT93 cells or 3x10⁶ DT6606 cells were subcutaneously implanted into the shaved right flanks of C57BL/6 male mice as described above. Once tumour volumes had reached approximately 100mm³, they were randomised into three groups and a dose of 1x10⁸ PFU of virus in 50 µl PBS or 50 µl PBS vehicle buffer control was injected as per the treatment schedules outlined in Table 3 (schedule 1 and 2). Tumour volumes were monitored via twice-weekly calliper measurement and mice were weighed weekly. Tumour growth was tracked twice weekly and animals were euthanized as governed by Home Office guidelines when tumour volumes approached 1000 mm³. There was a statistically significant reduction in tumour growth rate and prolonged survival favouring the VVL15-N1L agent upon treatment of the DT6606 flank tumour model (Fig13a-b), while in the CMT93 model there was no difference between tumour growth rates following IT administration of either viral agent, although both were significantly slower-growing than the PBS group (Fig 13 c-d).

### Example 10: Survival in orthotopic lung cancer following VVL15N1L administration

In order to assess whether the viruses were efficacious when administered intravenously, an orthotopic lung cancer model was utilised. 5x10⁶ LLC cells in 100µl PBS were injected into the tail veins of 7-week old female C57BL/6 mice.

Non contrast-enhanced CT scans of the lungs were used to assess the lung volumes of individual mice over a period of three weeks and any reduction used to extrapolate tumour burden. At a time, determined by the initial presence of tumour on CT, three doses of IV virus/PBS were administered as outlined in Table 3 (schedule 5). Mice were weighed twice weekly and were sacrificed if they showed signs of distress or if weight loss exceeded 20% of their maximal weight.

All mice developed tumours, with deaths occurring between 14 to 21 days, at which time thoracotomy confirmed extensive lung tumours. Tumours were initially apparent on CT between 4 and 7 days post-injection of LLC cells, thus day 5 post-injection was chosen as the start time for therapy.

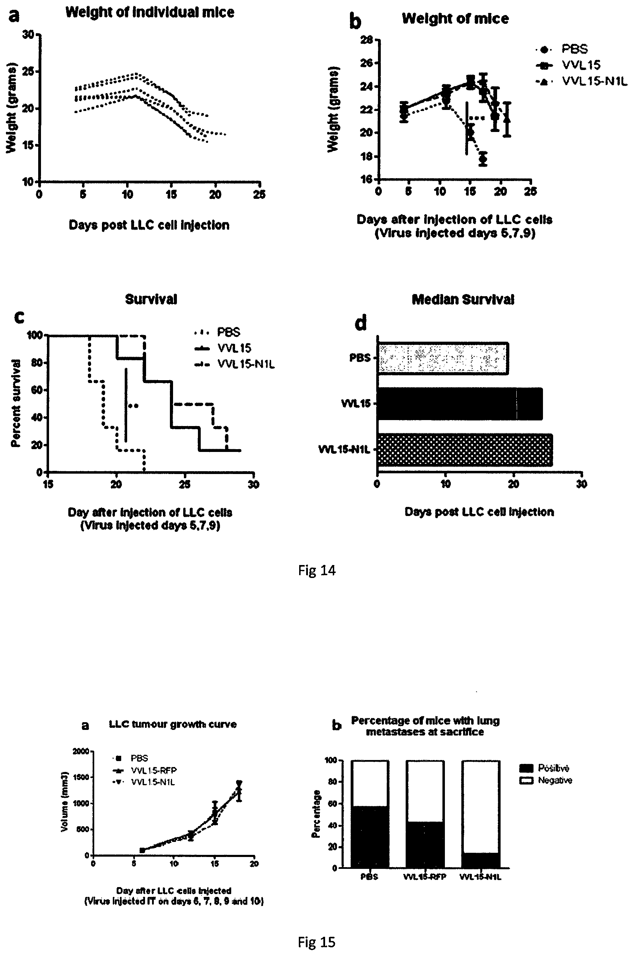

21 mice were administered with tail vein injections of 0.5x10⁶ LLC cells in 100µl serum-free DMEM. They were randomised into three groups and treatment (see Table 3, schedule 5) commenced from day 5. All mice in the PBS treatment group were symptomatic after 10 days post-injection of LLC cells as evidenced by weight loss and all had died by 21 days (Fig 14). The median survival was extended by 5 days with VVL15 and 6.5 days with VVL15-N1L viral treatments respectively, in comparison to the PBS group, although there was no statistically significant difference in survival between the viral groups.

### Example 11: Metastatic dissemination in lung cancer model following VVL15N1L administration

LLC is a very aggressive tumour model with a propensity to metastasise to the lung following subcutaneous flank injections. Indeed it has been reported that surgical excision of subcutaneously grown LLC tumour enhanced the rate of lung metastases, perhaps by the removal an angiogenesis inhibitor secreted by the primary.

To investigate whether IT injection of VV recombinants can reduce this metastatic rate, 1x10⁶ LLC cells were injected subcutaneously into the flanks of 7-week old female C57BL/6 mice. When tumour volumes were approximately 100 mm³, they were randomised into three groups. Injections of virus/ PBS were administered IT as per the treatment schedule in Table 3 (schedule 3). Tumours were monitored via calliper measurement until a group reached the end point of requiring sacrifice (approximately 17-20 days post implantation). All animals were euthanized at the same time, their lungs were harvested and any gross tumour deposits noted. Lung lobes were separated, fixed in 4% formalin, embedded in paraffin, stained with haematoxylin and eosin and sectioned through the largest cross-sectional dimension. For each lobe, slices were also performed above and below the largest cross section. All three sections were scrutinized for tumour deposits by a pathologist who was blinded to the treatment schedule.

There were no significant differences between groups with regards to tumour volumes at sacrifice (Fig 15a). However the percentage of mice with lung metastases at the endpoint of the experiment was 14, 43 and 57% for N1L, VVL15 and PBS groups respectively (Fig 15b). These figures were statistically different from each other. It is however difficult to draw inferences with such small numbers of mice per group (n=7) and the experiment would need to be repeated with larger sample sizes. However this result does suggest the possibility that even if VVL15N1L has no impact on the growth of an aggressive primary tumour, viral therapy may minimize dissemination. It could thus prove to be a good adjuvant therapy.

### Example 12: Efficacy of IL-12- and GM-CSF-armed VV15N1L

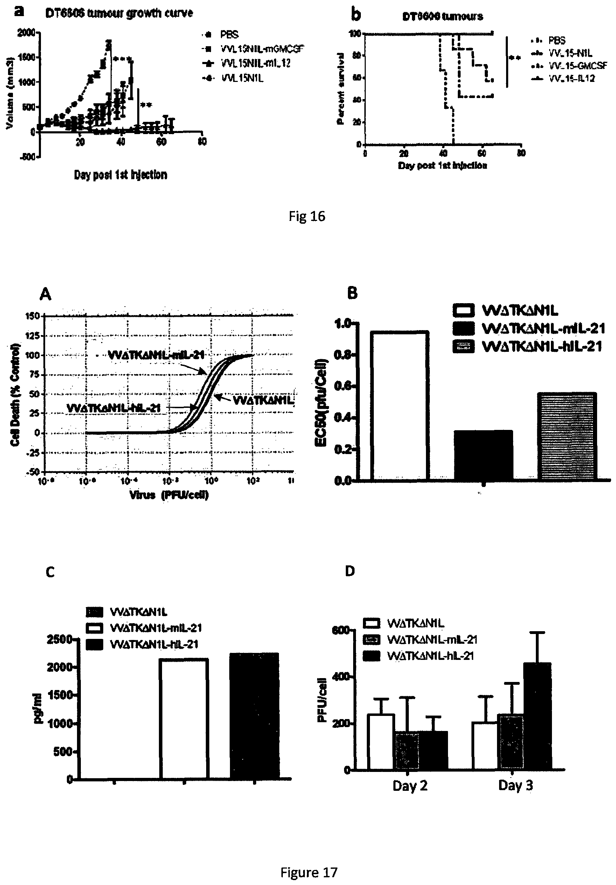

To enhance the antitumour efficacy of VVL15N1L, GM-CSF and IL-12 were inserted into the N1L region of the VVL15N1L vector. The potency of each of these recombinants was tested in vivo against a syngeneic DT6606 subcutaneous flank model (see Table 3, schedule 1). When tumour volumes reached an average of 100mm3, daily doses (5 in total) of 1x108 PFU of virus (VVL15-N1L, VVL15-N1L-mGMCSF or VVL15-N1L-mIL12) or the equivalent volume of vehicle buffer control (50ul of PBS) were injected IT (n=7 per group). Tumour growth was followed up via twice weekly calliper measurement (Fig 16a). The corresponding Kaplan Meir survival curves (Fig 16b) were based on the necessity for humane animal sacrifice when tumour volumes exceeded 1,000mm3. Figures 16 a-b demonstrate that the GMCSF transgene-armed virus alone was not significantly better than VVL15-N1L, however the IL12-armed virus demonstrated significant potency, leading to cures in 6/7 mice and 100% survival at the end of the experiment. These armed viruses will be tested in other models to establish the universality of this result.

### Examples 13 and 14: IL-21 constructs

### Materials and methods:

Murine pancreatic cancer model: DT6606 subcutaneous syngeneic tumours were established in male C57BL/6 mice. When tumours reached 5-6mm in diameter, PBS, VV-ΔTkΔN1L-mIL-21, VV-ΔTkΔN1L-hIL-21 or control virus VV-ΔTkΔN1L was administered intra-tumourally (5x10⁷pfu/injection) on day 1, 3, 7, 9 and 11. Tumour growth was measured twice weekly and animal survival was monitored. Survival data were compared using Prism® (GraphPad Software, CA, USA) and a log rank (Mantel Cox) test was used to determine significance of survival differences. Significance was determined using an unpaired students T test (*p<0.05; **p>0.01; ***p<0.001).

Syrian hamster cancer models: Syrian hamsters bearing HPD-1NR tumours - 1x10⁶ HPD-1NR cells were seeded by subcutaneous injection into the right flank of Syrian hamsters bearing HPD-1NR tumours. When tumours reached 313 mm³, PBS, VV-ΔTkΔN1L-mIL-21, VV-ΔTkΔN1L-hIL-21 or control virus VV-ΔTkΔN1L was administered intra-tumourally (5x10⁷pfu/injection) on day 1, 3, 7, 9 and 11. Tumours were measured twice weekly and animal survival was monitored. Survival data were compared using Prism® (GraphPad Software, CA, USA) and a log rank (Mantel Cox) test was used to determine significance of survival differences. Significance was determined using an unpaired students T test (*p<0.05; **p>0.01; ***p<0.001). Syrian hamster peritoneal cavity disseminated pancreatic cancer model - 1x10⁷ SHPC6 cells were seeded into the lower right peritoneal cavity of Syrian hamsters. Four days later, 10 hamsters per group were each injected intra-peritoneally (IP) with 500µl PBS, 2x10⁷ PFU of the different VVs on day 4, 6 and 8. The survival rates of hamsters were monitored. Survival data were compared using Prism® (GraphPad Software, CA, USA) and a log rank (Mantel Cox) test was used to determine significance of survival differences (* p<0.05, ** p<0.01, ***p<0.001).

### Example 13: Generation of IL-21 armed VV and assessment of anti-tumour potency in vitro

Human and mouse IL-21 cDNA sequences were inserted into the puc19N1L shuttle vector (as shown in Figure 3). The standard homologous recombinations were carried out in the TK-deleted backbone of Lister strain vaccinia virus (VVL15) by co-transfecting the resultant plasmid pShuttleN1L- mIL-21, or pShuttleN1L- hIL-21into CV-1 (African green monkey kidney) cells that were pre-infected with VVL15 at 0.05 PFU/cell. The transfected CV-1 cell lysates were subjected to plaque assay. Five plaque purification rounds were performed to propagate a single clonal virus and the modification was re-confirmed by PCR for the deletion of N1Lgene. The resultant viruses are named as VV-ΔTkΔN1L-mIL-21 and VV-ΔTkΔN1L-hIL-21. To test the potency of these viruses, a cell death assay (MTS assay) was used to detect the cytotoxicity of the three vaccinia viruses in a murine pancreatic cancer cell line (DT6606), which is derived from a mutant Ras-p53 transgenic pancreatic cancer model.

Figure 17 shows the assessment of IL-21 armed VV *in vitro.* Fig. 17 A &B show cytotoxicity of different oncolytic vaccinia viruses in a murine pancreatic cancer cell line. Cultures of the murine pancreatic cancer cell line DT6606 derived from Ras-p53 transgenic pancreatic cancer mice were infected with different viruses, and cell killing detected by MTS assay six days after viral infection. The curve of cell death induced by virus is shown in Fig. 17 A; the EC50 values (viral dose to kill 50% of cancer cells) were calculated (Fig. 17B). A higher EC50 value means that the virus has less potency. Fig. 17 C & D show detection of IL-21 expression and viral replication of different mutants of new generation of vaccinia virus in pancreatic cancer cells in vitro. Cultures of the murine pancreatic cancer cell line DT6606 derived from Ras-p53 transgenic pancreatic cancer mice were infected with different viruses, and IL-21 expression was detected by ELISA assay 24 hours after viral infection (Fig. 17C). The viral replication was detected by TCID50 assay is shown in Fig 17D. The experiments was triplicated.

As shown in Fig 17A & B, the new generation of oncolytic vaccinia virus (VV-ΔTkΔN1L) is still very effective in killing cancer cells; arming the virus with the cytokine IL-21 did not attenuate the virus, instead it increased the cytotoxicity against cancer cells by an (as yet) unknown mechanism (p<0.01).

In order to check whether the therapeutic gene IL-21 can be expressed in the virus-infected cancer cells and at what level, ELISA was used to detect the expression of the IL-21 protein from the VV-ΔTkΔN1L-mIL-21, VV-ΔTkΔN1L-hIL-21 and control virus VV-ΔTkΔN1L infected-pancreatic cancer cells (DT6606). As shown in Fig 17C, IL-21 was expressed at a very high level in the cells after infection with VV-ΔTkΔN1L-mIL-21, VV-ΔTkΔN1L-hIL-21, but the control virus VV-ΔTkΔN1L infection did not produce any IL-21 protein. The replication of IL-21 armed virus was not attenuated compare to backbone virus (Fig 17D).

### Example 14: Antitumour efficacy of IL-21 armed VV15N1L

To test whether IL21 can enhance the anti-tumour efficacy of VV-ΔTkΔN1L, the potency of each of these viruses (mouse IL21 or human IL21) was tested in vivo against a syngeneic DT6606 subcutaneous flank model.

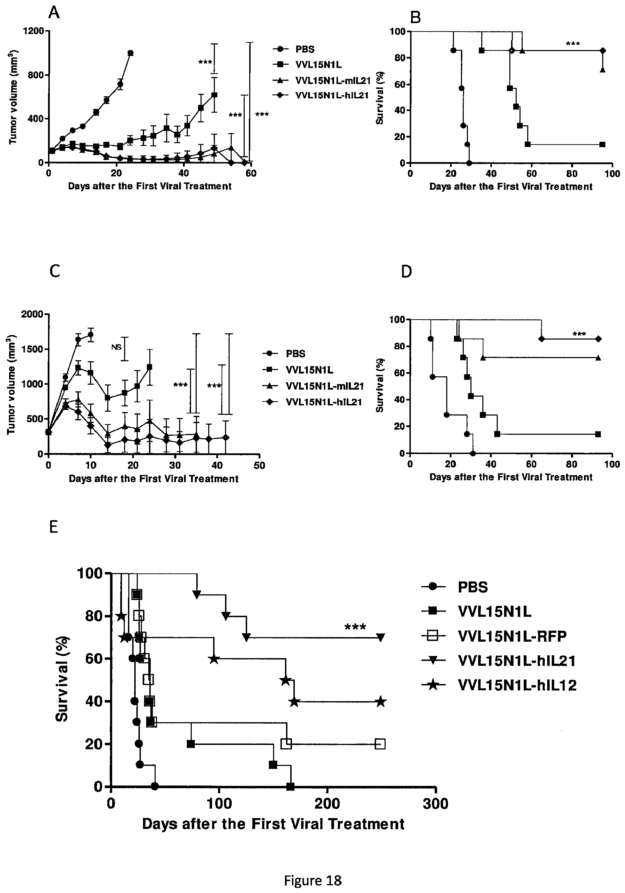

Figure 18 show the anti-tumour efficacy of VV-ΔTkΔN1L-mIL-21, VV-ΔTkΔN1L-hIL-21 and control virus VV-ΔTkΔN1L. Murine pancreatic cancer model - Tumour growth curve (Fig.18A) and the survival (Fig. 18B) of the mice treated with different agents are presented (n=7/group). Syrian hamsters bearing HPD-1NR tumours - Tumour growth curve (Fig. 18C) and the survival (Fig. 18D) of the mice treated with different agents are presented (n=7/group). In each case mean tumour size ± SEM are displayed until the death of the first hamster in each group and compared by one-way ANOVA with post-hoc Bonferroni testing. (Fig. 18E) shows the anti-tumour efficacy of the viral strains in the immunocompetent Syrian hamster peritoneal cavity disseminated pancreatic cancer model.

The IL21-armed virus demonstrated significant potency, regressed tumour growth (Fig18A) and marked prolonged the survival (Fig 18B) of the mice bearing pancreatic cancer. Given that human cytokines may function better in Syrian hamster than mouse, a subcutaneous Syrian hamster pancreatic cancer model was employed to evaluate the antitumour efficacy of IL21-armed virus. Strikingly the IL21-armed virus demonstrated significant potency, leading to cures in 6 of 7 animals and 86% survival at the end of the experiment (Figure 18C and D). Based on the antitumour efficacy in subcutaneous model, IL12 or IL21-armed VV demonstrate promising efficacy compared to control virus.

The major barrier for improving the survival of patients with pancreatic cancer is lacking effective therapeutic agent for advanced pancreatic cancer. To this end, a well-characterised Syrian hamster peritoneally disseminated pancreatic cancer model was used for assessment the feasibility, efficacy and safety of IL12 and IL21-armed VV. IL-12-armed VVLΔTKΔN1L showed induced severe toxicity after systemic delivery (Fig 18E). Strikingly, VVLΔTKΔN1L-IL21 has the highest therapeutic index for treatment of peritoneally disseminated pancreatic cancer, 70% of Syrian hamster were cured.

## Claims

1. A TK-deficient vaccinia virus comprising an inactivated N1L gene, wherein the N1L gene is inactivated by the insertion of a nucleic acid sequence encoding a heterologous polypeptide, and wherein the nucleic acid sequence comprises at least three vaccinia virus promoters, said vaccinia virus promoters are positioned in the same orientation which is opposite to that of the intact vaccinia virus ORF L024.

2. A TK-deficient vaccinia virus according to claim 1, wherein the vaccinia virus promoters are selected from the group consisting of mH5, H5, P7.5 and PE/L.

3. A TK-deficient vaccinia virus according to claim 1 or claim 2 wherein the polypeptide is a cytokine;

4. A TK-deficient vaccinia virus according to claim 3 wherein the polypeptide is selected from the group consisting of GM-CSF, IL-12 and IL-21.

5. A TK-deficient vaccinia virus according to any one of claims 1 to 4 for use in cancer therapy.

6. A composition comprising a TK-deficient vaccinia virus according to claims 1 to 4, optionally in the presence of a pharmaceutically acceptable carrier or excipient.

7. A composition according to claim 6 wherein the polypeptide is a cytokine selected from the group consisting of GM-CSF, IL-12 and IL-21.

8. A composition according to claim 6 or 7 for use in cancer therapy.

## Patentansprüche

1. TK-defizientes Vaccinia-Virus, umfassend ein inaktiviertes N1L-Gen, wobei das N1L-Gen durch Insertion einer Nukleinsäuresequenz, die ein heterologes Polypeptid kodiert, inaktiviert wird, und wobei die Nukleinsäuresequenz mindestens drei Vaccinia-Virus-Promotoren umfasst, wobei die Vaccinia-Virus-Promotoren in der gleichen Orientierung positioniert sind, die entgegengesetzt zu derjenigen des intakten Vaccinia-Virus-ORF L024 ist.

2. TK-defizientes Vaccinia-Virus nach Anspruch 1, wobei die Vaccinia-Virus-Promotoren ausgewählt sind aus der Gruppe bestehend aus mH5, H5, P7.5 und PE/L.

3. TK-defizientes Vaccinia-Virus nach Anspruch 1 oder Anspruch 2, wobei das Polypeptid ein Cytokin ist.

4. TK-defizientes Vaccinia-Virus nach Anspruch 3, wobei das Polypeptid ausgewählt ist aus der Gruppe bestehend aus GM-CSF, IL-12 und IL-21.

5. TK-defizientes Vaccinia-Virus nach einem der Ansprüche 1 bis 4 zur Verwendung in der Krebstherapie.

6. Zusammensetzung, umfassend ein TK-defizientes Vaccinia-Virus nach den Ansprüchen 1 bis 4, gegebenenfalls in Gegenwart eines pharmazeutisch verträglichen Trägers oder Exzipienten.

7. Zusammensetzung nach Anspruch 6, wobei das Polypeptid ein Cytokin ist, das ausgewählt ist aus der Gruppe bestehend aus GM-CSF, IL-12 und IL-21.

8. Zusammensetzung nach Anspruch 6 oder 7 zur Verwendung in der Krebstherapie.

## Revendications

1. Virus de la vaccine TK-déficient, comprenant un gène N1L inactivé, où le gène N1L est inactivé par l'insertion d'une séquence d'acide nucléique codant pour un polypeptide hétérologue, et où la séquence d'acide nucléique comprend au moins trois promoteurs du virus de la vaccine, lesdits promoteurs du virus de la vaccine étant positionnés selon la même orientation qui est opposée à celle de l'ORF L024 du virus de la vaccine intact.

2. Virus de la vaccine TK-déficient selon la revendication 1, où les promoteurs du virus de la vaccine sont choisis dans le groupe constitué par mH5, H5, P7.5 et PE/L.

3. Virus de la vaccine TK-déficient selon la revendication 1 ou la revendication 2, où le polypeptide est une cytokine.

4. Virus de la vaccine TK-déficient selon la revendication 3, où le polypeptide est choisi dans le groupe constitué par GM-CSF, IL-12 et IL-21.

5. Virus de la vaccine TK-déficient selon l'une quelconque des revendications 1 à 4, pour une utilisation dans une thérapie anticancéreuse.

6. Composition comprenant un virus de la vaccine TK-déficient selon les revendications 1 à 4, éventuellement en présence d'un véhicule ou excipient pharmaceutiquement acceptable.

7. Composition selon la revendication 6, dans laquelle le polypeptide est une cytokine choisie dans le groupe constitué par GM-CSF, IL-12 et IL-21.

8. Composition selon la revendication 6 ou 7, pour une utilisation dans une thérapie anticancéreuse.
